(19) Europäisches Patentamt
European Patent Office
Office européen des brevets

(11) Veröffentlichungsnummer: **0 100 019 B1**

# (12) EUROPÄISCHE PATENTSCHRIFT

(45) Veröffentlichungstag der Patentschrift: **19.06.91**

(51) Int. Cl.5: **C07B 37/00**, C07C 49/12, C07C 69/716, C07C 255/04, C07C 255/19, C07C 45/73, C07C 67/343, C07C 253/00

(21) Anmeldenummer: **83106778.0**

(22) Anmeldetag: **11.07.83**

Die Akte enthält technische Angaben, die nach dem Eingang der Anmeldung eingereicht wurden und die nicht in dieser Patentschrift enthalten sind.

## (54) Verfahren zur Herstellung von alpha-substituierten beta-Dicarbonyl-, beta-Cyancarbonyl- und beta-Dicyanverbindungen.

(30) Priorität: **22.07.82 DE 3227388**

(43) Veröffentlichungstag der Anmeldung: **08.02.84 Patentblatt 84/06**

(45) Bekanntmachung des Hinweises auf die Patenterteilung: **19.06.91 Patentblatt 91/25**

(84) Benannte Vertragsstaaten: **BE CH DE FR GB IT LI NL**

(56) Entgegenhaltungen:
**EP-A- 0 013 385**
**FR-A- 2 073 109**
**FR-A- 2 172 141**

**Chemical Abstracts, Band 84, Nr.15, 12.April 1976, Seite 529, Zusammenfassungs Nr. 105052f Columbus Ohio (US) H. Schmall et al.: "Reactions on aluminium oxide. 9. Knoevenagel condensation with aluminium oxide as catalyst." & Sci.Pharm. 1975, 43(3), 183-95**

**Krauch-Kunz, Reaktionen der organischen Chemie, 5. Auflage Hüthing Verlag, Heidelberg 1976, Seiten 77-79**

**Mathieu et al.: "Formation of C-C-Bonds, Band II Thieme Verlag, Stuttgart 1975, Seite 514**

**Org. Reactions, Vol. 15, 1978, Seiten 266-267**

(73) Patentinhaber: **BASF Aktiengesellschaft**
**Carl-Bosch-Strasse 38**
**W-6700 Ludwigshafen(DE)**

(72) Erfinder: **Gramlich, Walter, Dr.**
**Auf der Hoehe 11**
**W-6803 Edingen-Neckarhausen(DE)**
Erfinder: **Halbritter, Klaus, Dr.**
**Schwarzwaldstrasse 19**
**W-6800 Mannheim 1(DE)**
Erfinder: **Heilen, Gerd, Dr.**
**Schifferstadter Strasse 1b**
**W-6720 Speyer(DE)**

EP 0 100 019 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

## Beschreibung

Die vorliegende Erfindung betrifft ein verbessertes Verfahren zur Herstellung von α-substituierten β-Dicarbonyl-, β-Cyancarbonyl und β-Dicyanverbindungen der allgemeinen Formel I

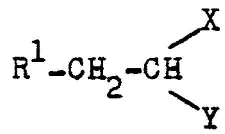

I

in der $R^1$ für Wasserstoff oder einen organischen Rest und X und Y für $-COOR^2$; $-CO-R^2$ oder -CN stehen, wobei $R^2$ einen organischen Rest bedeutet, durch Umsetzung eines Aldehyds II

$R^1$ - CHO   II

mit einer Verbindung III

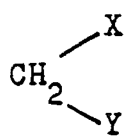

III

in Gegenwart eines Kondensationskatalysators, von Wasserstoff und einem Hydrierkatalysator in flüssiger Phase.

Die Umsetzung von Carbonylverbindungen, darunter von Aldehyden II,mit Verbindungen III zu Kondensationsprodukten des Typs IV

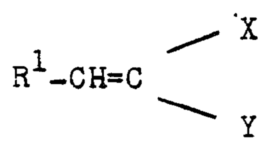

IV

ist allgemein als Knoevenagel-Reaktion bekannt (vgl. z.B. "Organikum" VEB Deutscher Verlag der Wissenschaften, 1962, Seite 442). Man nimmt sie in flüssiger Phase unter Verwendung verschiedener basischer oder saurer, meist homogener Katalysatoren unter fortlaufender Entfernung des entstehenden Reaktionswassers vor.

In der FR-A-2 172 141 wird empfohlen, die mit üblichen Katalysatoren bewirkte Knoevenagel-Kondensation von 2-Acetoxypropionaldehyd und Acetessigsäureethylester in Gegenwart wasserbindender Sustanzen auszuführen, unter denen Aluminiumoxid erwähnt ist.

Nach Chem. Abst. Band 84 (1976), Referat Nr. 105 052, diente für die Knoevenagel-Kondensation von Cyclohexanon mit Cyanessigsäureethylester basisches und saures Aluminiumoxid als Katalysator, jedoch sind hierbei die Gesamtausbeuten an den Zielprodukten Cyclohexyliden-cyanessigsäureethyl-ester und dem isomeren Cyclohex-1-enyl-cyanessigsäureethylester hierbei mit 10 - 15 % so gering, daß Aluminiumoxide als für die Knoevenagel-Reaktion ungeeignete Katalystoren anzusehen sind.

Weiterhin ist es aus der DE-A 20 60 443 bekannt, die Knoevenagelreaktion in Gegenwart von Wasserstoff und Hydrierkatalysatoren vorzunehmen und somit unmittelbar zu den Verbindungen I zu gelangen.

Schließlich ist es aus der GB-A 1 014 273 bekannt, die Aldolkondensation von Propionaldehyd unter hydrierenden Bedingungen mittels fester Kondensationskatalysatoren wie Oxiden und Phosphaten vorzunehmen, darunter solchen, wie sie im vorliegenden Falle anspruchsgemäß als Kondensationskatalysatoren vorgeschlagen werden, wobei man 2-Methylvaleraldehyd erhält. Da die Ausbeuten von 73 - 77 % hierbeirecht hoch sind, war hieraus und aus dem Referat cit. in Chem. Abstr. zu schließen, daß Aluminiumoxid die Aldolkondensation vorrangig begünstigt und daß die für eine Knoevenagel-Reaktion vorgesehenen

Aldehyde in einer Aldolkondensation vornehmlich mit sich selbst, nicht aber im Sinne der Knoevenagel-Reaktion reagieren.

Die gleiche Überlegung gilt für die EP-A-13 385, nach welcher Alkanole und Alkanone mittels Salzen oder Oxiden von Seltenen Erdmetallen erfolgreich (Ausbeuten 82-92 %) einer Aldolkondensation unterworfen werden.

Die für die Knoevenagelreaktion empfohlenen Kondensationskatalysatoren sind jedoch meist nur für bestimmte Fälle gut geeignet, so daß für die Herstellung verschiedener Verbindungen I jeweils gesonderte Vorschrift und Apparaturen erforderlich sind. Dies steht einer rationellen Herstellung verschiedener Verbindungen I, die als Spezialprodukte in der Regel jeweils nur in begrenzten Mengen benötigt werden, offensichtlich entgegen. Handelt es sich ferner um Verbindungen III bzw. I, in denen mindestens einer der Reste X und Y eine (-COOR$^2$)-Gruppe bedeutet, so findet häufig eine unerwünschte Decarboxylierung als Nebenreaktion statt, und schließlich bedingt die Verwendung homogener Katalysatoren einen entsprechenden Aufwand zu deren Abtrennung aus den entstehenden Reaktionsgemischen.

Der Erfindung lag daher die Aufgabe zugrunde, diesen Mängeln mittels eines universell anwendbaren heterogen katalysierten Verfahrens abzuhelfen, ohne daß hierfür Nachteile wie unerwünschte Nebenreaktionen oder Minderausbeuten in Kauf genommen werden müssen.

Demgemäß wurde gefunden, daß man die eingangs definierten Verbindungen I aus den ebenfalls eingangs definierten Verbindungen II und III in Gegenwart eines Kondensationskatalysators,von Wasserstoff sowie eines Hydriekatalysators in flüssiger Phase auf verfahrenstechnisch vorteilhafte Weise erhält, wenn man hierzu als Kondensationskatalysator ein Oxid oder Phosphat des Magnesiums, Aluminiums, Titans oder Zinks oder eines Seltenen Erdmetalls verwendet.

Es wurde weiterhin gefunden, daß man besonders gute Ergebnisse mit Aluminiumoxid als Kondensationskatalysator erhält.

Man kann die als Feststoffe vorliegenden Katalysatorkomponenten, den Hydrierkatalysator und den Kondensationskatalysator,im Prinzip getrennt voneinander einsetzen, jedoch empfiehlt sich in der Regel die Verwendung eines Mischkatalysators aus 0,1 bis 50 Gew.% der hydrierenden und 50 bis 99,9 Gew.% der kondensierenden Komponente.

Verwendet man Edelmetalle, insbesondere Palladium, als Hydrierkatalysatoren, stellt man die Mischkatalysatoren zweckmäßigerweise wie üblich in der Weise her, daß man den pulverförmigen Kondensationskatalysator mit einer wäßrigen Lösung eines Salzes eines der Edelmetalle zu einer Paste verarbeitet, wobei Menge und Konzentration der Salzlösung der gewünschten Katalysatorzusammensetzung entsprechen. Diese Paste wird sodann zu den Katalysatorteilchen verarbeitet, z.B. zu Kugeln von 1 bis 10 mm Durchmesser oder Zylindern von 0,5 bis 5 mm Durchmesser und 1 bis 10 mm Höhe, wonach man diese Teilchen trocknet und unter Wasserstoffatmosphäre auf 150 bis 1000 °C erhitzt, wobei die Edelmetallsalze zu den Metallen reduziert werden.

Ferner ist es nach den üblichen Techniken möglich, die Paste auf einen inerten Träger aus z.B. Kieselsäure aufzubringen und einen Trägerkatalysator herzustellen.

Bevorzugt wird Palladium als Hydrierkatalysator und Aluminiumoxid als Kondensationskatalysator. Unter den $Al_2O_3$-Modifikationen werden diejenigen mit einer großen spezifischen Oberfläche besonders bevorzugt, also die $\gamma$-Form (85 bis 400 m$^2$/g) und die $\eta$-Form (100 bis 600 m$^2$/g). $\alpha$-$Al_2O_3$ (40 bis 70 m$^3$/g) ist ebenfalls geeignet, erfordert aber etwas längere Reaktionszeiten.

Als Oxide und Phosphate von Seltenen Erdmetallen eignen sich vor allem solche des Cers, Praseodyms und Neodyms. Vorzugsweise verwendet man Mischungen aus 1 bis 10 Gew.% von Verbindungen der Seltenen Erdmetalle und dem Rest eines sonstigen definitionsgemäßen Oxids oder Phosphats, vor allem $Al_2O_3$.

Die für die erfindungsgemäße Umsetzung erforderliche Menge der Katalysatoren kann prinzipiell beliebig sein, da sie lediglich die Reaktionsgeschwindigkeit beeinflußt. Aus verfahrenstechnischen Gründen soll sie jedoch zweckmäßigerweise 0,4 kg pro kg des Gemisches aus II und III nicht überschreiten, und zur Erzielung wirtschaftlicher Raum-Zeit-Ausbeuten soll sie im allgemeinen nicht geringer sein als 0,01 kg pro kg des Gemisches aus II und III sein.

Sieht man vom festen Katalysator ab, nimmt man die Umsetzung in flüssiger Phase vor, d.h. sowohl die Ausgangsstoffe II und III als auch das Verfahrensprodukt sollen vollständig in flüssiger Phase vorliegen. Ist einer der Stoffe unter den Reaktionsbedingungen fest, verwendet man ein Lösungsmittel mit, und sind sie alle flüssig und ineinander mischbar, so erübrigt sich die Mitverwendung eines Lösungsmittels, sofern dessen Mitverwendung nicht aus sonstigen Gründen, z.B. weil die Ausgangsstoffe bereits in gelöster Form zur Verfügung stehen oder weil sich die Aufarbeitung dadurch einfacher gestaltet, zweckmäßig ist.

Als Lösungsmittel eignen sich inerte Flüssigkeiten, z.B. chlorierte Kohlenwasserstoffe wie Methylenchlorid, aromatische Kohlenwasserstoffe wie Toluol, Ether wie Tetrahydrofuran und Dioxan sowie $C_1$-$C_4$-

Alkohole, sofern letztere nicht in nennenswertem Ausmaß zu Umesterungen (X, Y = $-COOR^2$) Anlaß geben.

Je nach der Reaktivität der Ausgangsverbindungen II und III nimmt man die Umsetzung vorzugsweise im Druckbereich von 1 bis 50 bar $H_2$-Druck und im Temperaturbereich von 25 bis 180 °C vor. Im Regelfall arbeitet man bei 1 bis 10 bar $H_2$-Druck und 50 bis 150 °C.

II und III werden zweckmäßigerweise in äquimolaren Mengen eingesetzt. Liegt II im Überschuß vor, ist u.U. eine Selbstkondensation der überschüssigen Menge zu beobachten, und liegt III im Überschuß vor, so kann eine doppelte Addition an II unter Wasserabspaltung erfolgen.

Die durch die Anwesenheit von Wasser oder Überschüssen einer der Ausgangsstoffe bedingten Nebenreaktionen sind nach den bisherigen Beobachtungen jedoch von untergeordneter Bedeutung, da die Hauptreaktion (Addition von II an III, gefolgt von Dehydratisierung und Hydrierung) jeweils schneller verläuft als die Nebenreaktionen. Es ist daher lediglich darauf zu achten, daß die Zeiten für die Hauptreaktion nicht wesentlich überschritten werden, wie sich allerdings von selbst versteht.

Das erfindungsgemäße Verfahren, welches man nach den üblichen Techniken kontinuierlich oder diskontinuierlich vornehmen kann, ist von der Natur der Ausgangsverbindungen II und III grundsätzlich nicht abhängig.

Als Beispiele für die Aldehyde II seien genannt:

- aliphatische Aldehyde mit 1 bis 20 C-Atomen, vor allem $C_1$-$C_{20}$-Alkanale; Formaldehyd setzt man zweckmäßigerweise in fester polymerer Form, z.B. als Paraformaldehyd ein;
- cycloaliphatische Aldehyde, die sich von 5- oder 6-gliedrigen Ringverbindungen ableiten,
- araliphatische Aldehyde wie Phenylacetaldehyd,
- isocyclische aromatische Aldehyde, die sich vom Benzol oder von dessen höher kondensierten Ringsystemen ableiten, und
- heterocyclische aromatische Aldehyde wie die Pyridinaldehyde oder die Thiophenaldehyde.

Diese Aldehyde können als Substituenten z.B. Halogen, Cyangruppen, Nitrogruppen und zweifach substituierten Aminogruppen und als Heteroatome oder -atomgruppierungen Sauerstoff, Schwefel oder (-CO-O-) enthalten.

Enthalten die Aldehyde reaktive Gruppen oder Gruppierungen, so können sich diese u.U. verändern, jedoch wird hiervon das Prinzip der erfindungsgemäßen Reaktion nicht berührt. So können ungesättigte Aldehyde wie Acrolein bzw. die Derivate I dieser Aldehyde hydriert werden, jedoch hat man es nach den üblichen Hydriertechniken in der Hand, die Hydrierung zu begünstigen oder zu unterdrücken. Im allgemeinen bleibt der Charakter eines ungesättigten Restes $R^1$ jedoch erhalten, weil die durch die Kondensation entstehende Doppelbindung durch die Reste X und Y stark aktiviert und deswegen vorrangig hydriert wird.

Selbstverständlich gilt das Reaktionsprinzip auch für mehrwertige Aldehyde, z.B. für Terephthalaldehyd, so daß sich hier interessante Möglichkeiten für die Synthese von Verbindungen I ergeben, welche die (-CH-(X)Y)-Gruppierung zwei- oder mehrfach im Molekül enthalten.

Als Verbindungen III, deren charakteristisches Merkmal die aktivierte Methylengruppe zwischen den Substituenten X und Y ist, seien genannt:

- $C_1$-$C_{10}$-Alkylester der Malonsäure, vor allem deren Methyl- und Ethylester,
- Ester der Acylessigsäure, insbesondere deren $C_1$-$C_{10}$-Alkylester, vorzugsweise deren Methyl- und Ethylester,
- Acetylaceton und dessen höhere Homologe mit bis zu 10 C-Atomen in den Acylresten,
- $C_1$-$C_{10}$-Alkylester der Cyanessigsäure, insbesondere deren Methyl- und Ethylester,
- Acylessigsäurenitrile mit bis zu 10 C-Atomen im Acylrest, insbesondere Acetessigsäurenitril, und
- Malodinitril.

Ferner kommen auch solche Verbindungen III mit ($-CO-CH_2-CO-$)-Gruppierung in Betracht, in denen die Acylgruppen wie in Cyclopentan-1,3-dion zu einem Ring verbunden sind.

Durch das erfindungsgemäße Verfahren wird die Synthese der Verbindungen I erheblich vereinfacht. Die Verbindungen I dienen als Zwischenprodukte für weitere Synthesen, insbesondere von Arzneimitteln. So ist der aus n-Butyraldehyd und Cyanessigsäureethylester erhältliche n-Butylcyanessigsäureethylester eine Vorstufe für das bekannte Analgeticum Phenylbutazon.

## Beispiele

### Beispiele A bis J

Herstellung verschiedener Katalysatoren

Je 100 g eines pulvrigen, als Kondensationskatalysator dienenden Materials wurden mit 100 ml einer

wäßrigen Palladiumnitrat-Lösung zu einer Paste vermischt, aus der zylindrische Teilchen von 2 mm Durchmesser und 5 mm Höhe geformt wurden. Die Teilchen wurden getrocknet und unter Wasserstoffatmosphäre 6 Stunden lang auf 500 °C erhitzt, wobei das Pd zum Metall reduziert wurde. Die wäßrige Pd-nitrat-Lösung enthielt jeweils soviel des Salzes, wie der Zusammensetzung des Katalysators entsprach.

Tabelle 1 zeigt die Charakteristica der so hergestellten Katalysatoren:

Tabelle 1

| Katalysator | kondensierende Komponenten [g] | Pd-Gehalt Gew.% |
|---|---|---|
| A | $\gamma$-$Al_2O_3$, 100 | 2,00 |
| B | " | 0,75 |
| C | " | 0,50 |
| D | " | 1,00 |
| E | $\gamma$-$Al_2O_3$, 95; $Pr_2O_3$, 5 | 0,75 |
| F | $CePO_4$, 100 | 0,50 |
| G | $Dy_2O_3$, 100 | 1,00 |
| H | ZnO, 100 | 0,50 |
| I | $Zn_3(PO_4)_3$, 100 | 0,50 |
| J | $\alpha$-$Al_2O_3$, 100 | 10,00 |

Beispiele 1 bis 18

Herstellung verschiedener Verbindungen I

In einem Autoklaven wurden jeweils a g eines Aldehyds II, b g einer Verbindung III und c g eines Katalysators A bis J t Stunden lang unter einem Wasserstoffdruck von p bar auf T °C erhitzt. Die Mengen a und b verhielten sich jeweils äquimolar zueinander. Die übliche destillative Aufarbeitung des nach Abtrennung des Katalysators und des Reaktionswassers verbliebenen Reaktionsgemisches lieferte die gewünschte Verbindung I in einer Ausbeute von y %.

Die Versuchsbedingungen und -ergebnisse sind in Tabelle 2 zusammengestellt.

Tabelle 2

| Beispiel | Aldehyd II | [g] | Verbindung III | [g] | Kat. | [g] | t [h] | p [bar] | T [°C] | Verbindung I Sdp. [°C/mbar] | y [%] |
|---|---|---|---|---|---|---|---|---|---|---|---|
| 1 | n-Butanal | 535 | Cyanessigsäure-ethylester | 840 | A | 40 | 7 | 20 | 70 | n-Butylcyanessigsäureethylester 124-126/20 | 95 |
| 2 | Propanal | 52,2 | Acetylaceton | 90 | B | 7 | 6 | 20 | 75 | 3-Propylpentan--2,4-dion 102-110/20 | 93 |
| 3 | n-Butanal | 50,4 | Acetessigsäure-methylester | 81 | C | 6 | 8 | 35 | 80 | n-Butylacetessigsäuremethylester 102/10 | 92 |
| 4 | n-Octanal | 256 | Acetessigsäure-methylester | 232 | C | 20 | 3<br>+ 3 | 15<br>25 | 70<br>80 | n-Octylacetessigsäuremethylester 93-95/0,3 | 90 |
| 5 | 2-Phenyl-propanal | 134 | Cyanessigsäure-ethylester | 113 | D | 20 | 2<br>+ 2<br>+ 2 | 20<br>35<br>45 | 50<br>90<br>130 | 2-Cyano-4-phenyl-pentansäureethyl-ester; 128-132/0,2 | 85 |
| 6 | Methoxy-acetaldehyd | 82 | Cyanessigsäure-ethylester | 113 | B | 10 | 2<br>+ 2<br>+ 2 | 15<br>20<br>25 | 50<br>75<br>100 | 2-Cyano-4-methoxy--buttersäureethyl-ester; 128-132/22 | 91 |
| 7 | Paraform-aldehyd | 150 | Malonsäure-diethylester | 800 | C | 50 | 2<br>+ 2<br>+ 2 | 15<br>35<br>35 | 70<br>96<br>110 | Methylmalonsäure-diethylester 101-108 / 30 | 83 |
| 8 | iso-Butanal | 57,6 | Cyanessigsäure-ethylester | 90 | E | 6 | 2<br>+ 2<br>+ 2 | 20<br>40<br>55 | 70<br>110<br>130 | 2-Cyano-4-methyl-pentansäureethyl-ester; 135/22 | 90 |

| Beispiel | Aldehyd II | [g] | Verbindung III | [g] | Kat. | [g] | t [h] | p [bar] | T [°C] | Verbindung I Sdp. [°C/mbar] | y [%] |
|---|---|---|---|---|---|---|---|---|---|---|---|
| 9 | n-Hexanal | 200 | Malonsäurediethylester | 320 | E | 25 | 2 + 2 + 2 | 25 45 55 | 70 110 130 | n-Hexylmalonsäurediethylester 91-100/0,3 | 78 |
| 10 | Acetaldehyd | 48,4 | Cyanessigsäureethylester | 113 | C | 20 | 6 | 50 | 40 | Ethylcyanessigsäureethylester 50-60 / 0,3 | 70 |
| 11 | n-Butanal | 720 | Acetylaceton | 1000 | C | 70 | 3 + 3 | 30 35 | 70 80 | n-Butylpentan-2,4-dion 100-105/28 | 89 |
| 12 | 3-Formylpinan 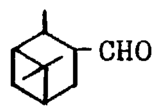 | 332 | Cyanessigsäureethylester | 226 | B | 50 | 3 + 3 | 35 40 | 70 75 | Pin-3-yl-methylcyanessigsäureethylester 110 / 0,1 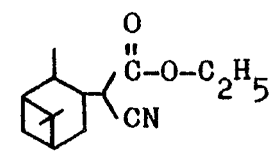 | 90 |
| 13 | Propanal | 29 | Malodinitril | 33 | F | 5 | 8 | 50 | 35 | n-Propylmalodinitril | 70 |
| 14 | n-Butanal | 50,4 | Acetessigsäuremethylester | 81 | G | 6 | 8 | 35 | 80 | n-Butylacetessigsäuremethylester | 80 |
| 15 | n-Butanal | 535 | Cyanessigsäureethylester | 840 | H | 40 | 7 | 20 | 70 | n-Butylcyanessigsäureethylester | 80 |
| 16 | n-Butanal | 535 | Cyanessigsäureethylester | 840 | I | 40 | 7 | 20 | 70 | n-Butylcyanessigsäureethylester | 82 |

| Beispiel | Aldehyd II | [g] | Verbindung III | [g] | Kat. | [g] | t [h] | p [bar] | T [°C] | Verbindung I Sdp. [°C/mbar] | y [%] |
|---|---|---|---|---|---|---|---|---|---|---|---|
| 17 | n-Butanal | 535 | Cyanessigsäure-ethylester | 840 | J | 50 | 12 | 1 | 70 | n-Butylcyanessigsäureethylester | 90 |
| 18 | Propanal | 29 | Cyclohexan-1,3-dion in 20 ml Methanol | 56 | C | 3 | 10 | 40 | 60 | n-Propylcyclohexan-2,6-dion | 75 |

## Ansprüche

1. Verfahren zur Herstellung von α-substituierten β-Dicarbonyl-, β-Cyancarbonyl- und β-Dicyanverbindungen der allgemeinen Formel I

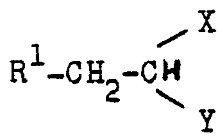

I

in der $R^1$ für Wasserstoff oder einen organischen Rest und X und Y für $-COOR^2$; $-CO-R^2$ oder -CN stehen, wobei $R^2$ einen organischen Rest bedeutet, durch Umsetzung eines Aldehyds II

$R^1$ - CHO II

mit einer Verbindung III

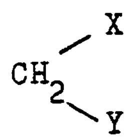

III

in Gegenwart eines Kondensationskatalysators, von Wasserstoff sowie eines Hydrierkatalysators in flüssiger Phase, dadurch gekennzeichnet, daß man als Kondensationskatalysator ein Oxid oder Phosphat des Magnesiums, Aluminiums, Titans, Zinks oder eines Seltenen Erdmetalls verwendet.

2. Verfahren nach Anspruch 1, dadurch gekennzeichnet, daß man einen Mischkatalysator aus dem Material des Kondensationskatalysators und einem Edelmetall der VIII. Gruppe des Periodensystems verwendet.

3. Verfahren nach Anspruch 2, dadurch gekennzeichnet, daß man als Edelmetall Palladium verwendet.

4. Verfahren nach den Ansprüchen 1 bis 3, dadurch gekennzeichnet, daß man als Kondensationskatalysator $\gamma$-$Al_2O_3$ oder $\eta$-$Al_2O_3$ verwendet.

## Claims

1. A process for the preparation of an $\alpha$-substituted $\beta$-dicarbonyl, $\beta$-cyanocarbonyl or $\beta$-dicyano compound of the formula I

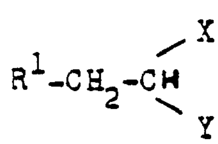

I

where $R^1$ is hydrogen or an organic radical and X and Y are $-COOR^2$, $-CO-R^2$ or -CN, where $R^2$ is an organic radical, by reacting an aldehyde II

$R^1$ - CHO II

with a compound III

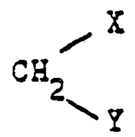

III

in the presence of a condensation catalyst, hydrogen and a hydrogenation catalyst in the liquid phase, wherein an oxide or phosphate of magnesium, aluminum, titanium, zinc or a rare earth metal is used as the condensation catalyst.

**2.** A process as claimed in claim 1, wherein a mixed catalyst of the material of the condensation catalyst and a noble metal of group VIII of the periodic table is used.

**3.** A process as claimed in claim 2, wherein palladium is used as the noble metal.

**4.** A process as claimed in claim 1 or 2 or 3, wherein $\gamma$-$Al_2O_3$ or $\eta$-$Al_2O_3$ is used as the condensation catalyst.

## Revendications

**1.** Procédé de préparation de composés du type $\beta$-dicarbonyle, $\beta$-cyanocarbonyle et $\beta$-dicyano, $\alpha$-substitués, de la formule générale I

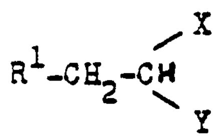

I

dans laquelle $R^1$ représente un atome d'hydrogène ou un radical organique et X et Y représentent $-COOR^2$, $-CO-R^2$ ou -CN, où $R^2$ représente un radical organique, par la réaction d'un aldéhyde de la formule II

$R^1$ - CHO II

avec un composé de la formule III

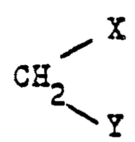

III

en présence d'un catalyseur de condensation, d'hydrogène, comme aussi d'un catalyseur d'hydrogénation en phase liquide, caractérisé en ce que l'on utilise, à titre de catalyseur de condensation, un oxyde ou un phosphate du magnésium, de l'aluminium, du titane, du zinc ou d'un métal des terres rares.

**2.** Procédé suivant la revendication 1, caractérisé en ce que l'on utilise un cocatalyseur constitué de la matière du catalyseur de condensation et d'un métal noble appartenant au huitième groupe du système périodique des éléments.

**3.** Procédé suivant la revendication 2, caractérisé en ce que l'on utilise le palladium à titre de métal noble.

**4.** Procédé suivant les revendications 1 à 3, caractérisé en ce que l'on utilise le $\delta$-$Al_2O_3$ ou le $\eta$-$Al_2O_3$ à titre de catalyseur de condensation.